(19) Europäisches Patentamt
European Patent Office
Office européen des brevets

(11) EP 2 806 849 B1

(12) EUROPEAN PATENT SPECIFICATION

(45) Date of publication and mention of the grant of the patent:
**08.02.2017 Bulletin 2017/06**

(21) Application number: **12812204.1**

(22) Date of filing: **14.12.2012**

(51) Int Cl.:
***A61K 8/35*** *(2006.01)* ***A61K 8/49*** *(2006.01)*
***A61K 8/97*** *(2017.01)* ***A61Q 17/04*** *(2006.01)*

(86) International application number:
**PCT/EP2012/075597**

(87) International publication number:
**WO 2013/110409 (01.08.2013 Gazette 2013/31)**

# (54) A PHOTOSTABLE SUNSCREEN COMPOSITION

LICHTSTABILE SONNENABSCHIRMUNGSZUSAMMENSETZUNG

COMPOSITION D'ÉCRAN SOLAIRE PHOTOSTABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.01.2012 PCT/CN2012/000126**
**05.03.2012 EP 12158056**

(43) Date of publication of application:
**03.12.2014 Bulletin 2014/49**

(73) Proprietors:
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
- **Unilever PLC**
**London, Greater London EC4Y 0DY (GB)**
Designated Contracting States:
**CY GB IE MT**

(72) Inventors:
- **SHUKLA, Ravi Kant**
**Whitefield**
**Bangalore 560 066 (IN)**
- **GADGIL, Vijay Ramchandra**
**Whitefield**
**Bangalore 560 066 (IN)**
- **BANDYOPADHYAY, Punam**
**Whitefield**
**Bangalore 560 066 (IN)**
- **LI, Lin**
**Shanghai**
**Changning District 200335 (CN)**

(74) Representative: **Corsten, Michael Allan**
**Unilever PLC**
**Unilever Patent Group**
**Colworth House**
**Sharnbrook**
**Bedford MK44 1LQ (GB)**

(56) References cited:
**WO-A1-91/11989**
**US-A1- 2003 091 665**
**US-A1- 2010 292 318**

- **DATABASE GNPD [Online] MINTEL; June 2002 (2002-06), "Biologie + Skin Optimiser", XP002682050, Database accession no. 152945**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 806 849 B1

## Description

### Technical Field

The invention relates to a photo-stable sunscreen composition comprising a sunscreen stabilizer. The sunscreen stabilizer may be synthetically prepared or extracted from a plant.

### Background of the invention

Solar radiation includes ultraviolet (UV) radiation, wavelength of which is between 200 nm and 400 nm. Exposure of skin to UV-A (320 to 400 nm) and UV-B (290 to 320 nm) causes various problems like reddening of the skin, localized irritation, sunburn, melanoma and formation of wrinkles. UV radiation is also known to cause damage to hair. Therefore, it is desirable to protect the skin and other keratinous substrates of the human body from the harmful effects of both UV-A and UV-B radiation.

Cosmetic compositions comprising sunscreen agents are used to protect the skin against UV radiation. The most commonly used UV-A sunscreen is of the dibenzoylmethane class. They are often used along with UV-B sunscreens to get wide spectrum sunscreen protection. It has been reported that stability of dibenzoylmethane compounds in sunscreen compositions is low when applied to the skin and exposed to solar radiation. The stability is poorer when an oil-soluble UV-B organic sunscreens especially from the class of cinnamic acid is included.

Synthetic sunscreen stabilizers have been reported to solve this problem e.g. as disclosed in EP 0514491 (L'Oreal, 1991) and more recently in US2009/039322 (Hallstar).

While the above references are directed to improving stability of sunscreen containing compositions, it is desirable to use stabilizers that are available in nature or extractable from natural sources like plants that the consumers consider to be milder and therefore is expected to have less irritation and allergenic potential when applied on the skin. Stabilisers which are available in nature or extractable from natural sources is also expected to be widely available and therefore can be provided to the consumers at low cost. Topical compositions comprising extracts from plant material are known for various purposes.

US200303091665 discloses a topical cosmetic composition for improving the aesthetic appearance of skin comprising: a blend of neem seed cell broth and one or more botanical ingredients selected from the group consisting of *Salvia miltiorrhiza* extract, pomegranate fruit extract, grape seed extract, cucumber extract, carrot extract, rosemary extract, Iris root extract, white birch extract, and Laminaria algae extract.

US20100292318 discloses a selectively purified tanshinone compounds containing extract from the root of a Salvia spp comprising Cryptotanshinone, Dihydrotanshinone, Tanshinone I, and Tanshinone IIA, the extract and formulations thereof have been found to exhibit anti-microbial properties.

These publications have not disclosed use of actives claimed in the present invention or natural extracts comprising the actives for stabilizing UV-A sunscreens.

It is thus an object of the present invention to obviate the drawbacks of the prior art and provide a highly photo-stable sunscreen composition while ensuring prolonged efficacy of the UV-A organic sunscreen used therein.

Another object of the present invention is to achieve the above object while additionally keeping costs low.

### Summary of the Invention

The present invention provides for a photostable sunscreen composition comprising

a. 0.1 % to 10 % by weight dibenzoylmethane or its derivative;

b. a UV-B organic sunscreen selected from the class consisting of cinnamic acid, salicylic acid, diphenyl acrylic acid or derivatives thereof

c. 0.0001 to 5% by weight a compound of the formula;

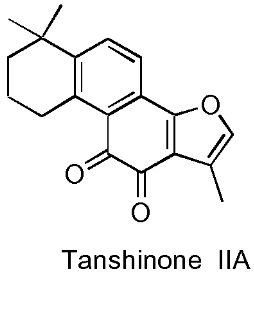

Tanshinone IIA

and
d. a cosmetically acceptable base.

## Detailed description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. It is more preferably a leave-on product. The composition of the present invention can be in the form of a liquid, lotion, cream, foam, scrub, gel, or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

An advantage of the present invention is that inclusion of the sunscreen stabilizer Tanshinone IIA ensures that the UV-A sunscreen does not get degraded in the presence of UV radiation thereby providing UV-protection for long period of time to the substrate of interest.

The photostable sunscreen composition of the invention comprises UV-A sunscreen dibenzoylmethane or its derivative; a sunscreen stabliser which is the compound Tanshinone IIA and a cosmetically acceptable base.

The sunscreen composition of the invention comprises a UV- A sunscreen which is a dibenzoylmethane or its derivatives. Dibenzoylmethane derivatives are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'- methoxy dibenzoylmethane or 2,6-dimethyl-4-tert-butyl-4'-methoxydibenzoylmethane. The most preferred dibenzoylmethane derivative is 4-tert.-butyl-4'-methoxydibenzoylmethane. The composition of the invention preferably comprises 0.1 to 10%, more preferably 0.2 to 5%, further more preferably, 0.4 to 3%, by weight dibenzoylmethane or a derivative thereof based on total weight weight of the composition and including all ranges subsumed therein.

Tanshinone IIA has the structure

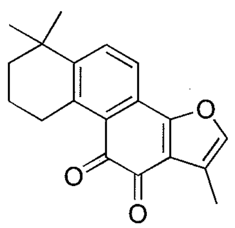

Tanshinone IIA

Tanshinone IIA is a compound of the family of orthoquinones.

Tanshinone IIA is preferably present in 0.0005 to 3 %, more preferably 0.001 to 2 % by weight of the sunscreen composition.

The stable sunscreen composition of the invention preferably also comprises 0.0001 to 2% a compound of the

formula

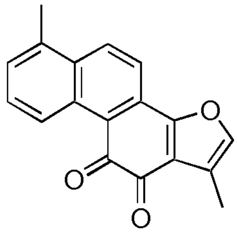

Tanshinone I

Tanshinone I is a compound of the family of orthoquinones.

Tanshinone I is preferably present in 0.001 to 0.5% by weight of the sunscreen composition.

According to a preferred aspect of the present invention, the Tanshinone IIA and/ or Tanshinone I is present by including in the composition 0.1 to 10% by weight an extract of a plant comprising higher than 50 weight % diterpenoid orthoquinones. The extract is preferably included at 0.1 to 5 % by weight of the composition. The extract preferably comprises higher than 1.0 % by weight diterpenoid orthoquinones. The plant is preferably *Salvia miltiorrhiza.*

*Salvia miltiorrhiza (S. miltiorrhiza,* also know as danshen), is a perennial plant which grows mainly in China and Japan in the forests, hillsides and along stream banks. This plant is highly valued in Traditional Chinese Medicine system (TCM). It is used as an internal medicine for promoting blood circulation to remove blood stasis, regulating menstruation to relieve pain, cooling the blood to relieve carbuncle, clearing away heat from the heart and tranquilizing the mind.

*Botanical classification of Salvia miltiorrhiza Bunge is as follows;*

| | |
|---|---|
| Kingdom: | Plantae |
| Sub kingdom: | Angiosperms |
| Order: | Lamiales |
| Family: | Lamiaceae |
| Genus: | *Salvia* |
| Species: | S. *miltiorrhiza* |

The sunscreen composition comprises a UV-B organic sunscreen selected from the class of cinnamic acid, salicylic acid, diphenyl acrylic acid or derivatives thereof. UV-B sunscreens which are commercially available and useful for inclusion in the composition of the invention are Octisalate™, Homosalate™, NeoHelipan™, Octocrylene™, Oxybenzone™ or Parsol MCX™. The UV-B sunscreen is most preferably 2-ethyl-hexyl-4-methoxy cinnamate which is commercially available as Parsol MCX. The UV-B organic sunscreen is preferably included in 0.1 to 10%, more preferably 0.1 to 7 % by weight of the composition. It has been observed that presence of an organic UV-B sunscreen like 2-ethylhexyl-4-methoxy cinnamate causes further rapid degradation of the UV-A dibenzoylmethane sunscreen in the presence of UV radiation. The presence of the Tanshinone IIA compound is found to be very efficacious in stabilizing the sunscreen composition even when UV-B sunscreens are present.

The composition of the invention comprises a cosmetically acceptable base. The cosmetically acceptable base preferably comprises 1 to 25% fatty acid. The cosmetically acceptable bases are such as to have a product in preferably a cream, lotion, gel or emulsion format. A more preferred format is a cream, further more preferably a vanishing cream. Vanishing cream base is one which comprises 3 to 25%, more preferably 5 to 20% fatty acid. The base preferably comprises 0.1 to 10%, more preferably 0.1 to 3% soap. $C_{12}$ to $C_{20}$ fatty acids are especially preferred in vanishing cream bases, further more preferred being $C_{14}$ to $C_{18}$ fatty acids. In creams, the fatty acid is preferably substantially a mixture of stearic acid and palmitic acid. Soaps in the vanishing cream base include alkali metal salt of fatty acids, like sodium or potassium salts. The soap is preferably the potassium salt of the fatty acid mixture. The fatty acid in vanishing cream base is often prepared using hystric acid which is substantially (generally about 90 to 95%) a mixture of stearic acid and palmitic acid. Thus, inclusion of hystric acid and its soap to prepare the vanishing cream base is within the scope of the present invention. It is particularly preferred that the composition comprises at least 6%, preferably at least 10%, more preferably at least 12% fatty acid. The cosmetically acceptable base is usually from 10 to 99.9%, preferably from 50 to 99% by weight of the composition. Another preferred base is a lotion. Lotions generally comprise 1 to 20% fatty acid. The cosmetically acceptable base preferably includes water. Water is preferably included in 35 to 90%, more preferably 50 to 85%, further more preferably 50 to 80% by weight of the composition.

Other useful sun-protective agents e.g. inorganic sun-blocks may be preferably used in the present invention. These include, for example, zinc oxide, iron oxide, silica, such as fumed silica, or titanium dioxide. The total amount of

sun block that is preferably incorporated in the composition according to the invention is from 0.1 to 5% by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

When the composition is prepared by including an extract of *Salvia miltiorrhiza* comprising the Tanshinone IIA in the desired concentration a preferred process comprises the steps of

(a) extracting the plant *Salvia miltiorrhiza* with a hydroalcoholic solution comprising 5 to 95 wt% ethanol to prepare a hydro-alcoholic extract; and
(b) fractionating the desired extract from said hydro-alcoholic extract using ethyl acetate.

The hydroalcoholic solution preferably comprises 10 to 99 %, more preferably 90 to 98 % ethanol by weight of the hydroalcoholic solution. The root part of the plant *Salvia miltiorrhiza* is preferably used for extraction. The plant and the hydro-alcoholic solution is preferably contacted in a weight ratio of 1:5 to 1:50. The hydroalcoholic extract and the ethyl acetate are preferably contacted in a weight ratio of 1:10 to 1:30 to fractionate the desired extract. The process is preferably carried out at a temperature in the range of 20 to 60°C, preferably 25 to 45 °C.

S. *miltiorrhiza,* for use in the present invention, was obtained from Unilever R&D Lab, in Shanghai China.

The invention is now further described by way of the following non-limiting examples.

## Examples

Examples 1 to 4: Photostability of the composition of the invention as compared to compositions outside the invention

Several compositions as shown in table 1 were prepared. The procedure used to prepare the extract of *Salvia miltiorrhiza* (example 3) was as follows:

Aqueous ethanolic extract of *S. miltiorrhiza* (50g), was re-dissolved in distilled water (1 L) at 45-50°C by stirring for 15 minutes. The resultant suspended solution was extracted with ethyl acetate (200 ml X 5). Ethyl acetate layer was collected over $Na_2SO_4$ and dried on rota evaporator. About 10 g of a dried powder was obtained.

Table 1

| Example | Example 1, wt% | Example 2, wt% | Example 3, wt% | Example 4, wt% |
|---|---|---|---|---|
| Stearic acid | 17.00 | 17.00 | 17.00 | 17.00 |

(continued)

| Example | Example 1, wt% | Example 2, wt% | Example 3, wt% | Example 4, wt% |
|---|---|---|---|---|
| Potassium hydroxide | 0.57 | 0.57 | 0.57 | 0.57 |
| Glycerine | 1.00 | 1.00 | 1.00 | 1.00 |
| Titanium dioxide | 0.90 | 0.90 | 0.90 | 0.90 |
| Cetyl alcohol | 0.53 | 0.53 | 0.53 | 0.53 |
| Parsol 1789 | 0.40 | 0.40 | 0.40 | 0.40 |
| Parsol MCX | 0.75 | 0.75 | 0.75 | - |
| Isopropyl Mysristate | 0.75 | 0.75 | 0.75 | 0.75 |
| Silicone oil 200/300 cst | 0.33 | 0.33 | 0.33 | 0.33 |
| Niacinamide | 1.25 | 1.25 | 1.25 | 1.25 |
| Titanium dioxide | 0.90 | 0.90 | 0.90 | 0.90 |
| Octocrylene | - | 0.5 | - | - |
| *Salvia miltiorrhiza* extract | - | - | 0.5# | - |
| Water | To 100 | To 100 | To 100 | To 100 |
| # The *Salvia miltiorrhiza* extract used was analysed to contain ~2.0% Tanshinone IIA. The concentration of Tanshinone IIA in the extract was determined using chromatographic separation over silica gel in Combiflash, and eluted with gradual increment of ethyl acetate in hexane. | | | | |

Procedure to measure photostability of dibenzoylmethane compound:

10 mg of the composition comprising dibenzoylmethane based UVA sunscreen was spread on an area of 2 $cm^2$ of a glass slides. Several such glass slides were prepared. The slides were exposed to sunlamp. After time period of 0 (blank, no exposure), 30, 60 and 120 minutes of UV exposure, the slides were removed. The compositions in the exposed and unexposed slides were extracted in methanol, the volume made up to 25 ml in a volumetric flask and the absorbance was measured using a Perkin-Elmer spectrophotometer. From the absorbance, the amount of sunscreen remaining was calculated. The sunscreen in unexposed slide was taken to be 100% and all others were quantified with respect to it using equation given below.

$$Q_t = \frac{\mathcal{A}_t}{\mathcal{A}_0} X100$$

Where $Q_t$ is the percentage of sunscreen present after t minutes of sun exposure, $A_0$ and $A_t$ are the absorbance of extracted solutions after sun exposure for zero time and time of 't' minutes.

The stability of the UV-A sunscreen was measured for the compositions of Examples 1 to 3 and the data is summarized in table 2.

Table 2:

| Time of Exposure (minutes) | Example 1 | Example 2 | Example 3 | Example 4 |
|---|---|---|---|---|
| 0 | 100 | 100 | 100 | 100 |
| 30 | 40 | 69 | 87 | 80 |
| 60 | 31 | 52 | 78 | 58 |
| 120 | 14 | 36 | 66 | 36 |

The data in table 2 indicates that use of Tanshinone IIA rich fraction (example 3) significantly enhances efficacy of a sunscreen composition comprising UVA and UVB sunscreen. The efficacy is vastly superior as compared to well

known chemical stabilizer Octocrylene (example 2).

Examples 5 and 6: Demonstration of pure Tanshinione IIA as a photostabiliser for Parsol-1789

The efficacy of Tanshinone IIA for photostabilisation of UV-A sunscreen Parsol-1789 (a dibenzoyl methane compound) was demonstrated in chloroform solution at 5 ppm concentration. Example 5 is a solution of Parsol - 1789 alone at 5ppm while example 6 contains both Parsol-1789 and Tanshinone IIA at 5 ppm each. In example 5, pure Tanshinone IIA was extracted from the plant *Salvia miltiorrhiza* using the following procedure:

Hydroalcoholic extract of Salvia miltiorrhiza was first prepared by contacting root of the plant *Salvia miltiorrhiza* (50g) in 95% ethanol/water mixture at a weight ratio of root to mixture of 1:20. The extract was then re-dissolved in distilled water (1 L) at 45-50°C by stirring for 15 minutes. The desired fraction was fractionated with ethyl acetate and water. The ethyl acetate fraction was dried in a rotary evaporator. This fraction (ethyl acetate) was adsorbed on a silica gel column chromatographic equipment (from Combiflash). The Column was then eluted with gradual increment of ethyl acetate in n-hexane upto 5% level. Using this procedure Tanshinone-IIA was prepared in pure form.

Degradation profile of Parsol-1789 on exposure to UV radiation was carried out similar to examples 1 to 3. The data is presented in table 3.

Table 3

| Time of Exposure (minutes) | Example 5 | Example 6 |
|---|---|---|
| 0 | 100 | 100 |
| 30 | 58 | 77 |
| 60 | 47 | 70 |

The data in table 3 indicates that Tanshinone IIA is capable of stabilizing UV-A sunscreen of the dibenzoylmethane class.

The present invention thus provides for stable sunscreen composition with prolonged efficacy of the UV-A sunscreen used. All this is achieved using actives extractable from natural sources.

## Claims

**1.** A photostable sunscreen composition comprising

a. 0.1 % to 10 % by weight dibenzoylmethane or its derivative selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyldibenzoylmethane, 2-methyl-5-isopropyl-4'-methoxydibenzoylmethane, 2-methyl-5-tert-butyl-4'-methoxydibenzoyl methane, 2,4-dimethyl-4'- methoxy dibenzoylmethane or 2,6-dimethyl-4-tert-butyl-4'-methoxy-dibenzoylmethane;
b. a UV-B organic sunscreen selected from the class consisting of cinnamic acid, salicylic acid, diphenyl acrylic acid or derivatives thereof selected from Octisalate™ (octyl salicylate), Homosalate™ (3,3,5-trimethyleyclohexyl 2-hydroxybenzoate), NeoHelipan™ (a range of organic UV filters including ethylhexyl methoxycinnamate and ethylhexyl salicylate), Octocrylene™ (2-ethylhexyl 2-cyano-3,3-diphenyl-2-propenoate) or Parsol MCX™ (2-ethylhexyl-4-methoxycinnamate).
c. 0.0001 to 5% by weight a compound of the formula;

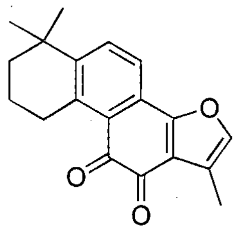

Tanshinone IIA

and
d. a cosmetically acceptable base.

2. A composition as claimed in claim 1 additionally comprising 0.0001 to 2% a compound of the formula

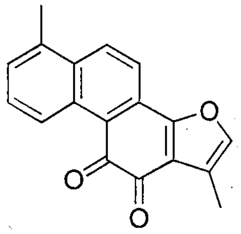

Tanshinone I

3. A composition as claimed in claim 1 or claim 2 wherein said Tanshinone IIA is present by including in said composition 0.1 to 10% by weight an extract of a plant comprising higher than 50 weight % diterpenoid orthoquinones.

4. A composition as claimed in claim 3 wherein said plant is *Salvia miltiorrhiza.*

5. A composition as claimed in any one of the preceding claims wherein said UV-B organic sunscreen is present in 0.1% to 10% by weight of the composition.

6. A composition as claimed in any one of the preceding claims, wherein said UV-B sunscreen is 2-ethyl-hexyl-4-methoxy cinnamate.

7. A composition as claimed in any one of the preceding claims wherein the cosmetically acceptable base comprises 1 to 25 % by weight fatty acid.

8. A composition as claimed in any one of the preceding claims wherein the cosmetically acceptable base comprises 0.1 to 10% by weight soap.

9. A composition as claimed in any one of the preceding claims 4 to 8 wherein said extract is prepared by a process comprising the steps of

(a) extracting the plant *Salvia miltiorrhiza* with a hydroalcoholic solution comprising 5 to 95 wt% ethanol to prepare a hydro-alcoholic extract; and
(b) fractionating the desired extract from said hydro-alcoholic extract using ethyl acetate.

**Patentansprüche**

1. Lichtstabile Sonnenschutzzusammensetzung, umfassend

a. 0,1 bis 10 Gewichts-% Dibenzoylmethan oder dessen Derivate, ausgewählt aus 4-tert.-Butyl-4'-methoxydibenzoylmethan, 2-Methyldibenzoylmethan, 4-Methyldibenzoylethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan oder 2,6-Dimethyl-4-tert.-butyl-4'-methoxydibenzoylmethan,
b. ein organisches UV-B-Sonnenschutzmittel, ausgewählt aus der Klasse, bestehend aus Zimtsäure, Salicylsäure, Diphenylacrylsäure oder den Derivaten hiervon, ausgewählt aus Octisalate^WZ (Octylsalicylat), Homosalate^WZ (3,3,5-Trimethylcyclohexyl-2-hydroxybenzoat), NeoHelipan^WZ (ein Bereich von organischen UV-Filtern, einschließlich Ethylhexylmethoxycinnamat und Ethylhexylsalicylat), Octocrylene^WZ (2-Ethylhexyl-2-cyano-3,3-diphenyl-2-propenoat) oder Parsol MCX^WZ (2-Ethylhexyl-4-methoxycinnamat),
c. 0,0001 bis 5 Gewichts-% einer Verbindung der Formel

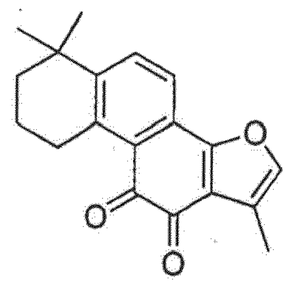

Tanshinone IIA

und
d. einen kosmetisch annehmbaren Träger.

**2.** Zusammensetzung, wie im Anspruch 1 beansprucht, die zusätzlich 0,0001 bis 2% einer Verbindung der Formel

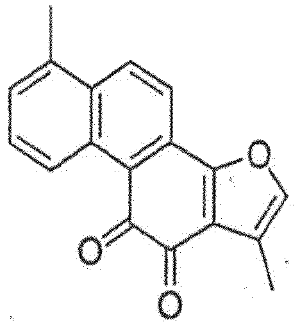

Tanshinone I

umfasst.

**3.** Zusammensetzung, wie im Anspruch 1 oder im Anspruch 2 beansprucht, worin Tanshinone IIA vorliegt, indem der Zusammensetzung 0,1 bis 10 Gewichts-% eines Extraktes einer Pflanze einverleibt wird, die mehr als 50 Gewichts-% Diterpenoidorthochinone enthält.

**4.** Zusammensetzung, wie im Anspruch 3 beansprucht, wobei es sich bei der Pflanze um die *Salvia miltiorrhiza* handelt.

**5.** Zusammensetzung, wie in irgendeinem der vorhergehenden Ansprüche beansprucht, worin das organische UV-B-Sonnenschutzmittel in 0,1 bis 10 Gewichts-% der Zusammensetzung vorliegt.

**6.** Zusammensetzung, wie in irgendeinem der vorhergehenden Ansprüche beansprucht, wobei das UV-B-Sonnenschutzmittel 2-Ethylhexyl-4-methoxycinnamat ist.

**7.** Zusammensetzung, wie in irgendeinem der vorhergehenden Ansprüche beansprucht, worin der kosmetisch annehmbare Träger 1 bis 25 Gewichts-% Fettsäure umfasst.

**8.** Zusammensetzung, wie in irgendeinem der vorhergehenden Ansprüche beansprucht, wobei der kosmetisch annehmbare Träger 0,1 bis 10 Gewichts-% Seife umfasst.

**9.** Zusammensetzung, wie in irgendeinem der vorhergehenden Ansprüche 4 bis 8 beansprucht, wobei der Extrakt durch ein Verfahren hergestellt wird, das die Schritte umfasst

(a) Extrahieren der Pflanze *Salvia miltiorrhiza* mit einer wässrig-alkoholischen Lösung, die 5 bis 95 Gewichts-% Ethanol enthält, um einen wässrigalkoholischen Extrakt zu gewinnen, und
(b) Fraktionieren des gewünschten Extrakts von dem wässrig-alkoholischen Extrakt unter Verwendung von Ethylacetat.

## Revendications

**1.** Composition solaire photostable comprenant

a. de 0,1 % à 10 % en poids de dibenzoylméthane ou de son dérivé choisi parmi le 4-tert-butyl-4'-méthoxydibenzoylméthane, le 2-méthyldibenzoylméthane, le 4-méthyl-dibenzoyl-éthane, le 4-isopropyldibenzoyl-méthane, le 4-tert-butyldibenzoylméthane, le 2,4-diméthyldibenzoylméthane, le 2,5-diméthyldibenzoylméthane, le 4,4'-diisopropyl-dibenzoylméthane, le 2-méthyl-5-isopropyl-4'-méthoxydibenzoylméthane, le 2-méthyl-5-tert-butyl-4'-méthoxy-dibenzoylméthane, le 2,4-diméthyl-4'-méthoxydibenzoylméthane ou le 2,6-diméthyl-4-tert-butyl-4'-méthoxy-dibenzoylméthane ;
b. un écran solaire contre les UV-B choisi dans la classe constituée par l'acide cinnamique, l'acide salicylique, l'acide diphénylacrylique ou des dérivés de ceux-ci choisis parmi Octisalate™ (salicylate d'octyle), Homosalate™ (2-hydroxybenzoate de 3,3,5-triméthyleycyclohexyle), NeoHelipan™ (un gamme de filtres UV organiques comprenant du méthoxycinnamate d'éthylhexyle et du salicylate d'éthylhexyle), Octocrylene™ (2-cyano-3,3-diphényl-2-propénoate de 2-éthylhexyle), ou Parsol MCX™ (4-méthoxycinnamate de 2-éthylhexyle) ;
c. de 0,0001 à 5 % en poids d'un composé de formule :

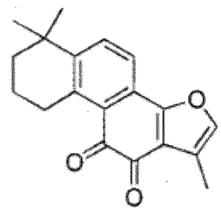

Tanshinone IIA

et
d. une base cosmétiquement acceptable.

**2.** Composition selon la revendication 1, comprenant en outre de 0,0001 à 2 % d'un composé de formule :

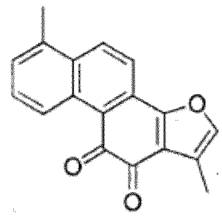

Tanshinone I.

**3.** Composition selon la revendication 1 ou la revendication 2, dans laquelle ladite Tanshinone IIA est présente en incluant dans ladite composition de 0,1 à 10 % en poids d'un extrait d'une plante comprenant plus de 50 % en poids d'orthoquinones diterpénoïdes.

**4.** Composition selon la revendication 3, dans laquelle ladite plante est Salvia miltiorrhiza.

**5.** Composition selon l'une quelconque des revendications précédentes, dans laquelle ledit écran solaire organique

contre les UV-B est présent à hauteur de 0,1 % à 10 % en poids de la composition.

**6.** Composition selon l'une quelconque des revendications précédentes, dans laquelle ledit écran solaire contre les UV-B est le cinnamate de 2-éthyl-hexyl-4-méthoxy.

**7.** Composition selon l'une quelconque des revendications précédentes, dans laquelle la base cosmétiquement acceptable comprend de 1 à 25 % en poids d'un acide gras.

**8.** Composition selon l'une quelconque des revendications précédentes, dans laquelle la base cosmétiquement acceptable comprend de 0,1 à 10 % en poids d'un savon.

**9.** Composition selon l'une quelconque des revendications 4 à 8, dans laquelle ledit extrait est préparé par un procédé comprenant les étapes consistant à

(a) extraire la plante *Salvia miltiorrhiza* avec une solution hydroalcoolique comprenant de 5 à 95 % en poids d'éthanol pour préparer un extrait hydroalcoolique ; et
(b) fractionner l'extrait souhaité à partir dudit extrait hydroalcoolique en utilisant de l'acétate d'éthyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0514491 A, L'Oreal
- US 2009039322 A, Hallstar
- US 200303091665 A
- US 20100292318 A

### Non-patent literature cited in the description

- The CTFA Cosmetic Ingredient Handbook. 1992